# EUROPÄISCHE PATENTANMELDUNG

(11) **EP 0 811 847 A2**
(43) Veröffentlichungstag der Anmeldung: **10.12.1997**
(21) Anmeldenummer: 97108986.7
(22) Anmeldetag: 04.06.1997
(51) Int. Cl.: G01N 33/558, G01N 33/94

(54) **Verfahren zum Nachweis von Analyten auf einer Oberfläche**

(30) Priorität: 05.06.1996 DE 19622503
(71) Anmelder: BOEHRINGER MANNHEIM GMBH, 68298 Mannheim (DE); SECURETEC GmbH, 85521 Ottobrunn (DE)
(72) Erfinder: Droste, Holger, Dr., 69214 Eppelheim (DE); Linke, Sandra, 68165 Mannheim (DE); Aberl, Franz, Dr., 85402 Kranzberg (DE); Bonenberger, Johannes, Dr., 80637 München (DE); Sachs, Hans, Dr., 89134 (DE); Goerlach-Graw, Ada, Dr., 67229 Grosskarlbach (DE)

(57) **Zusammenfassung**

Gegenstand der Erfindung ist ein Verfahren zum Nachweis eines Analyten in Körperflüssigkeit mit einem Testkit, das umfaßt,
a) einen Teststreifen aus einem oder mehreren kapillaraktiven chromatographiefähigen flächenförmigen Materialien in Fluidkontakt zueinander mit
   - einer Elutionsmittelaufgabezone an einem Ende und einer Zielzone am anderen Ende,
   - einer Abfangzone zwischen Elutionsmittelaufgabezone und Zielzone, in der ein Abfangreagenz immobilisiert ist, das fähig ist, entweder den Analyten, einen spezifischen Analytbindungspartner oder einen markierten Bindungspartner spezifisch zu binden und
   - einer Konjugatzone, zwischen Elutionsmittelaufgabezone und Abfangzone, die einen wanderungsfähigen markierten Bindungspartner enthält, der fähig ist, entweder den Analyten oder den spezifischen Analytbindungspartner oder das Abfangreagenz spezifisch zu binden, wobei die Bindung des markierten Bindungspartners zu einem detektierbaren Signal in der Abfangzone oder in der Zielzone führt, das die Anwesenheit des Analyten anzeigt,
b) ein Probennahmeelement separat zur Teststreifenoberfläche
c) eine Vorrichtung, die bewirkt, daß das Probennahmeelement mit der Teststreifenoberfläche zwischen Elutionsmittelauftragszone und Konjugatzone oder in der Konjugatzone selbst kontaktiert werden kann,
   dadurch gekennzeichnet, daß mit dem Probennahmeelement Körperflüssigkeit von einer Körperregion aufgenommen und untersucht wird.

Besonders geeignet ist das Verfahren zum Nachweis von Drogen.

## Beschreibung

Die Erfindung betrifft ein Verfahren zum Nachweis eines Analyten auf einer Oberfläche durch Aufnehmen des Analyten von der Oberfläche mit einem Probennahmeelement, Elution des Analyten von dem Probennahmeelement und Nachweis des Analyten in der Elutionsflüssigkeit durch eine immunologische Nachweisreaktion.

Neben dem Nachweis von Analyten in Probeflüssigkeiten, wie z. B. Blut, Urin, Speichel, kommt insbesondere in der Kriminalistik dem Nachweis von Analyten auf festen Oberflächen, wie z. B. auf Möbeln, Gepäck usw., eine wachsende Bedeutung zu. Speziell in der Drogenfahndung ist es ein wünschenswertes Ziel, auch sehr geringe Drogenkontaminationen von Gegenständen einfach und schnell nachweisen zu können. Je sensitiver dabei ein Nachweisverfahren ist, um so weniger Analytmenge, die eine bestimmte Oberfläche kontaminiert, muß dabei auf dieser Oberfläche erfaßt werden.

Um beliebige Oberflächen auf Analyte, insbesondere auf Drogen, zu untersuchen, sind verschiedene spezielle Nachweistechniken bekannt, bei denen zuerst der Analyt durch Abwischen einer Oberfläche gesammelt und nach Elution von der benutzten Wischoberfläche immunologisch nachgewiesen wird. Bei dem "Illicit Substance Detector" der Firma Westinghouse, Baltimore, USA (Security Management, Vol. 37/8, Seite 12 - 15, 1993), wird mit einer genoppten Kunststoffoberfläche eine zu untersuchende Oberfläche abgewischt und die Droge über ein in eine Einwegkarte integriertes Verfahren mit drei Reagenzlösungen nachgewiesen (DE-A-4341862). Das Testergebnis wird mit einem optischen Lesegerät ausgewertet. Das immunologische Nachweisprinzip beruht auf der Inhibierung einer Latex-Agglutinationsreaktion durch die nachzuweisende Droge. Die untere Nachweisgrenze wird mit einem Mikrogramm angegeben. Nachteilig neben der recht hohen Nachweisgrenze ist, daß die Nachweisreaktion durch ein mechanisches Auspressen der drei Flüssigkeitsreservoirs gestartet werden muß. Zudem kann das Meßergebnis nur mit einer optischen Lesehilfe und nicht mit dem Auge ausgewertet werden.

Die Firma Roche Diagnostics stellt einen Testkit auf Kokain aus Urinproben nach demselben aufwendig zu handhabenden immunologischen Nachweisprinzip her. Die Nachweisempfindlichkeit liegt im Bereich von 0,2 µg/ml.

Der "Accupress-Kit" der Firma Thermetics, Woburn, USA (Security Management, Vol. 37/8, Seite 12 - 15, 1993), besteht aus einem speziell beschichteten Reagenzträger und drei Gefäßen mit Reagenzlösung. Bei diesem Test wird mit einem Wattestäbchen die zu untersuchende Oberfläche abgewischt und das Wattestäbchen anschließend mit Puffer ausgewaschen. Neben der vergleichbar unempfindlichen Nachweisgrenze von nicht unter einem µg ist die Handhabung von drei verschiedenen Reagenzlösungen umständlich und zieht auch Fehlermöglichkeiten nach sich.

In EP-A-0 699 906 ist ein Testkit beschrieben, mit dem Analyte von Oberflächen abgewischt und nach gewiesen werden, wobei diese Oberflächen durch direkten Kontakt bei der Handhabung mit solchen Analyten kontaminiert wurden.

Aufgabe der Erfindung war es, ein Verfahren zum Nachweis von Analytkontaminationen von Oberflächen, insbesondere mit Drogenspuren, bereitzustellen, das in einfacher Weise und ohne technische Hilfsmittel durchgeführt werden kann. Insbesondere sollte die Nachweisgrenze für den Analyt deutlich unter einem µg absolut, möglichst unter 100 ng liegen.

Gelöst wird die Aufgabe durch ein Verfahren, wie es in den Ansprüchen charakterisiert ist.

Es wurde überraschend gefunden, daß der Testkit, der in EP-A-0 699 906 beschrieben ist, auch erfolgreich für die Untersuchung von Körperregionen eingesetzt werden kann, die nicht bei der Handhabung mit der nachzuweisenden Substanz durch direkten Kontakt kontaminiert wurden. Im wesentlichen handelt es sich um solche Körperpartien, die Körperflüssigkeiten wie Schweiß, Speichel, Tränen, Urin etc. ausscheiden oder sie tragen, wie Mund, Mundschleimhaut, Zunge, Achse, Augen, Genitalbereich etc.

Gegenstand der Erfindung ist ein Verfahren zum Nachweis eines Analyten in Körperflüssigkeit mit einem Testkit, das umfaßt
a) einen Teststreifen aus einem oder mehreren kapillaraktiven chromatographiefähigen flächenförmigen Materialien in Fluidkontakt zueinander mit
   - einer Elutionsmittelaufgabezone an einem Ende und einer Zielzone am anderen Ende,
   - einer Abfangzone zwischen Elutionsmittelaufgabezone und Zielzone, in der ein Abfangreagenz immobilisiert ist, das fähig ist, entweder den Analyten, einen spezifischen Analytbindungspartner oder einen markierten Bindungspartner spezifisch zu binden und
   - einer Konjugatzone, zwischen Elutionsmittelaufgabezone und Abfangzone, die einen wanderungsfähigen markierten Bindungspartner enthält, der fähig ist, entweder den Analyten oder den spezifischen Analytbindungspartner oder das Abfangreagenz spezifisch zu binden, wobei die Bindung des markierten Bindungspartners zu einem detektierbaren Signal in der Abfangzone oder in der Zielzone führt, das die Anwesenheit des Analyten anzeigt,
b) ein Probennahmeelement separat zur Teststreifenoberfläche
c) eine Vorrichtung, die bewirkt, daß das Probennahmeelement mit der Teststreifenoberfläche zwischen Elutionsmittelauftragszone und Konjugatzone oder in der Konjugatzone selbst kontaktiert werden kann,
   dadurch gekennzeichnet, daß mit dem Probennahmeelement Körperflüssigkeit von einer Körperregion aufgenommen und untersucht wird.

Dieses Verfahren umfaßt das Abwischen des Analyten von einer Körperoberfläche mit einem Probennahmeelement aus einem Wischmaterial, Elution des Analyten von dem Probennahmeelement und Nachweis des Analyten in der Elutionsflüssigkeit durch eine immunologische Nachweisreaktion, dadurch gekennzeichnet, daß
a) mit dem Probennahmeelement die auf den Analyt zu untersuchende Oberfläche abgewischt wird,
b) das Probennahmeelement mit der flächigen Oberfläche eines kapillaraktiven chromatographiefähigen Teststreifens, der eine Elutionsmittelaufgabezone an einem Ende und eine Zielzone am anderen Ende des Streifens enthält, in einem Bereich zwischen beiden Zonen kontaktiert wird,
c) Elutionsflüssigkeit auf die Elutionsmittelaufgabezone aufgegeben wird, die durch Kapillarkraft zur Zielzone an der Kontaktstelle mit der Probennahmefläche vorbeiwandert, wobei Analyt auf dem Probennahmeelement von der Elutionsflüssigkeit aufgenommen wird und
d) der Analyt in der Zielzone aufgrund einer immunologischen Bindungsreaktion gemessen wird.
Daneben umfaßt das erfindungsgemäße Verfahren auch die Gewinnung von Probenmaterial, vorzugsweise aus dem Mund, durch intensives Inkontaktbringen des Probennahmeelements mit der entsprechenden Körperregion. Das Probennahmeelement kann dabei ein schwammartiges Material sein, welches vom Probanden beispielsweise durch Kauen mit Probenflüssigkeit, insbesondere Speichel, gefüllt wird.

Der Teststreifen für das erfindungsgemäße Verfahren kann aus einem einzigen chromatographiefähigen streifenförmigen Material oder bevorzugt aus mehreren auf einer Basisschicht im wesentlichen nebeneinander angeordneten kapillaraktiven Flächen aus gleichen oder verschiedenen Materialien bestehen, die in Fluidkontakt zueinander stehen, so daß sie eine Flüssigkeitstransportstrecke bilden, entlang derer eine Flüssigkeit, durch Kapillarkräfte getrieben, von der Elutionsmittelaufgabezone zu der Zielzone strömt.

Als chromatographisches Material kommen alle flüssigkeitsabsorbierenden, porösen oder kapillaraktiven Materialien in Frage, z.B. Cellulose und deren Derivate, Glasfasern und Vliese und Gewebe aus künstlichen oder natürlichen Materialien.

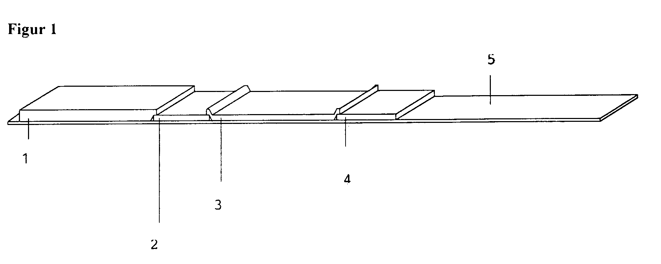

Es können in dem erfindungsgemäßen Verfahren verschiedene immunologische Testführungen angewendet werden mit denen der Analyt aufgrund einer oder mehrerer immunologischer Bindungsreaktionen nachgewiesen wird. In einer bevorzugten Ausführungsform (Figur 1) enthält ein für die Erfindung geeigneter Chromatographie-Teststreifen gegebenenfalls auf einer Trägerfolie (5) zwischen der Elutionsmittelaufgabezone (1) und der Zielzone (4) eine Abfangzone (3), die ein Abfangreagenz in immobilisierter Form enthält, das fähig ist, entweder den Analyten, einen spezifischen Bindungspartner des Analyten oder einen markierten Bindungspartner spezifisch zu binden. Vor der Abfangzone enthält der Teststreifen bevorzugt eine Konjugatzone (2), die einen wanderungsfähigen markierten Bindungspartner enthält, der fähig ist, entweder den Analyten, einen spezifischen Bindungspartner des Analyten oder das Abfangreagenz in der Abfangzone spezifisch zu binden.

Bei dem "spezifischen Bindungspartner des Analyten" handelt es sich um einen wanderungsfähigen, unmarkierten Analytbindungspartner mit einer Bindungsstelle für das Abfangreagenz. Wenn ein solcher Bindungspartner in dem Immunoassay verwendet wird, so kann dieser vor oder in der Konjugatzone oder bevorzugt zwischen Konjugatzone und Abfangzone aufgebracht sein. Zusätzlich kann sich nach der Zielzone noch weiteres flüssigkeitsabsorbierendes Material anschließen, das Flüssigkeit nach einer Wanderung durch die einzelnen Zonen aufnimmt.

Je nach Art des Immunoassays, der in dem erfindungsgemäßen Verfahren angewandt werden soll, liegen in den verschiedenen Zonen unterschiedliche Bindungspartner vor: Bei einem Sandwich-Immunoassay liegen bevorzugt in der Konjugatzone ein markierter Analytbindungspartner nicht-immobilisiert vor. Dieser bildet mit dem Analyten einen Komplex, der entweder durch das Abfangreagenz durch dessen Bindung an den Analyten gebunden wird, oder ein zweiter, unmarkierter, frei wanderungsfähiger Analytbindepartner mit einer spezifischen Bindungsstelle für das Abfangreagenz bildet zuerst einen Sandwich-Komplex mit dem Analyten, der mit Hilfe der spezifischen Bindungsstelle des Bindungspartners in der Abfangzone gebunden wird. Bevorzugt wird dabei die Markierung des Komplexes in der Abfangzone gemessen. In diesem Fall sind Abfangzone und Zielzone identisch.

Bei einem kompetitiven Test befindet sich bevorzugt in der Konjugatzone ein markiertes Analyt-Analogon, das bei Anwesenheit des Analyten entweder um die Bindungsstellen des Abfangreagenzes in der Abfangzone konkurriert (in diesem Fall ist das Abfangreagenz ein Analytbindungspartner) oder, wenn ein zusätzlicher wanderungsfähiger Analytbindungspartner vorhanden ist, um dessen Bindungsstelle konkurriert. Komplexe von markiertem Analyt-Analogon und wanderungsfähigem Bindungspartner werden dann über eine spezifische Bindungsstelle, z. B. Biotin, in der Abfangzone gebunden. In diesem Falle ist das Abfangreagenz ein Bindungspartner des wanderungsfähigen Analytbindungspartners, z. B. Streptavidin. Bevorzugt wird bei diesem Verfahren die Markierung nicht in der Abfangzone, sondern in der Zielzone in Form der unkomplexierten Analyt-Analoga als Maß für die Anwesenheit des Analpen gemessen.

Bevorzugt wird das erfindungsgemäße Verfahren nach einem IEMA-analogen Testprinzip (auch "immunenzymometrisch-analoges" Testprinzip) durchgeführt. Die Durchführung von IEMA-Tests auf Teststreifen ist beispielsweise in EP-A-0 407 904, EP-A-0 353 570 oder DE OS 4024919 beschrieben.

In der Konjugatzone befinden sich markierte Bindungspartner für den Analyten im Überschuß. Nach chromatographischer Wanderung werden nicht an Analyt gebundene markierte Bindungspartner durch festphasengebundene Analyt-Analoga in der Abfangzone immobilisiert, während Komplexe aus Analyt und markiertem Bindungspartner in die Zielzone weiterchromatographieren. Die in die Zielzone gelangte Markierung kann als Maß für die Anwesenheit oder auch die Konzentration des Analyten gemessen werden.

Als Analyt dienen in der vorliegenden Erfindung alle immunologisch nachweisbaren Substanzen insbesondere Antigene und Haptene. Ganz besonders geeignet ist die vorliegende Erfindung zum Nachweis von Drogen, z. B. Kokain, Morphin, Heroin. Dabei kann der Analyt auf der abzuwischenden Oberfläche als Molekül oder partikulär oder an Partikel adsorbiert vorkommen.

Als Markierung kommen übliche Markierungen, wie Enzymmarkierung, Fluoreszenz-, Farbstoffmarkierung, in Frage. Bevorzugt ist Direktmarkierung, insbesondere Metallmarkierung, ganz besonders bevorzugt Goldmarkierung. Diese hat den Vorteil, daß direkt mit dem Auge das Testergebnis abgelesen werden kann.

Als Bindungspartner für den Analyten kommen insbesondere Antikörper und Antikörperfragmente in Betracht.

Falls ein wanderungsfähiger unmarkierter Analytbindungspartner zusätzlich zum Abfangreagenz benutzt wird, trägt dieser eine Bindungsstelle für das Abfangreagenz. Für diese Bindungsstelle kommen alle spezifischen Bindungspartner eines spezifischen Bindungspaares in Betracht, z. B. Lektine, Antikörper, Antigene, bevorzugt Biotin (das mit Streptavidin bindet). Die Markierung in der Abfangzone bzw. Zielzone kann über übliche Nachweismethoden erfolgen, z. B. reflektionsphotometrisch oder visuell. Insbesondere bei Metallmarkierung, z. B. Goldmarkierung, kann das Meßergebnis in einfacher Weise visuell abgelesen werden.

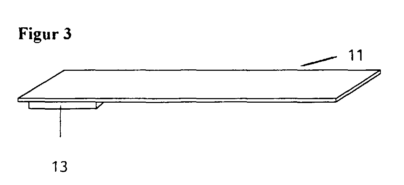

Zur Durchführung des erfindungsgemäßen Verfahrens wird mit einem Probennahmeelement über eine zu untersuchende Oberfläche vorteilhafterweise unter Anwendung eines leichten Drucks gewischt. Das Probennahmeelement dient dabei als Wischelement. Andererseits kann das Probennahmeelement als Schwamm gestaltet sein, der sich selbständig oder durch Kauen im Mund des Probanden mit Probenflüssigkeit füllt. Die zu untersuchende Körperregion trägt in der Regel Feuchtigkeit. Es ist vorteilhaft, wenn der Probennahmevorgang mehrmals wiederholt wird. Zur besseren Handhabung ist das Probennahmeelement (13) bevorzugt auf einem Träger (11) befestigt (Figur 3). Eine gute Wischleistung ergibt sich insbesondere bei einer hohen mechanischen Beanspruchbarkeit des Probennahmeelements. Besonders vorteilhaft hat es sich dafür erwiesen, wenn das Material des Probennahmeelements mit dem Träger (11) mit Ultraschall verschweißt wird.

Als Probennahmeelement können alle Materialien wie Kunststoffe, Gewebe, Vliese benutzt werden, auf denen Analyte, insbesondere Drogen haften bleiben und sich durch Wischen anreichern lassen. Andererseits sollten die anhaltenden Analyten bei Flüssigkeitskontakt wieder leicht desorbierbar sein. Der Fachmann kann ohne Aufwand solche Materialien auswählen. Bevorzugt sind saugfähige Materialien wie Vliese, Gewebe oder poröse Matrices wie Membranen oder Schwämme. Ganz besonders geeignet sind Vliese aus faserigen Materialien, wobei die Fasern im allgemeinen ungeordnet zusammengefügt sind, z.B. Papier oder Glasfaservliese. Die Probennahmeoberfläche ist bevorzugt trocken, kann aber auch angefeuchtet werden.

Werden rauhe Oberflächen wie z.B. anodisierte Aluminiumoberflächen oder andere angerauhte Metalloberflächen untersucht, wird bevorzugt eine angefeuchtete Probennahmeoberfläche eingesetzt. Geeignete Flüssigkeiten zur Anfeuchtung der Probennahmefläche sind primär Wasser und wäßrige Puffersysteme, die auf die nachfolgende Immunreaktion abgestimmt sein können. Dem Wasser oder den wäßrigen Puffern können Detergentien oder organische Lösungsmittel zugefügt sein. Als Detergentien können Tween 20, Tween 80, Octylglucosid, Polidocanol, Synperonic, z.B. F 68, oder auch zwitterionische Detergentien wie Cholamidopropansulfonat in einer Konzentration unter 5 Gew.- %, bevorzugt bei 0,01 Gew.-% bis 1 Gew.-% allein oder in Mischungen verwendet werden. Als organische Lösungsmittel können z.B. Dimethylsulfoxid, Glycerin oder Ethanol allein oder in Mischungen eingesetzt werden. Der Anteil an Lösungsmittel in der Flüssigkeit kann deutlich unter 30 Gew.-% betragen. Es ist aber auch möglich, organische Lösemittel oder Mischungen organischer Lösungsmittel ohne Wasser zu verwenden.

Die Befeuchtung der Probennahmefläche wird durch die Aufgabe von 1 - 50 µl der Flüssigkeit pro cm² , bevorzugt 3 - 20 µl / cm² z. B. durch Aufgabe mit einer Pipette oder einer automatischen Dosiervorrichtung erreicht. Alternativ kann die Befeuchtung auch durch kurzzeitigen Kontakt der Probennahmefläche mit einem feuchten Schwamm oder einer anderen feuchtigkeitsabgebenden Oberfläche erfolgen.

Die Befeuchtung der Probennahmefläche kann auch vorteilhaft so gelöst werden, daß die Flüssigkeit in mikroverkapselter oder blisterverpackter Form auf dem für das Wischen vorgesehenen Teil bereits vorgehalten wird. Wenn als Flüssigkeit Wasser oder wässrige Puffersysteme eingesetzt werden, wird die Verkapselung vorteilhaft durch Einschluß in wachsartige Substanzen, wie z.B. Paraffin, vorgenommen. Das Freisetzen der Flüssigkeit kann auf mechanischem Weg, z.B. durch Andrücken auf der zu untersuchenden Oberfläche, erfolgen.

Bevorzugt ist das Probennahmeelement ein flächenförmiges Erzeugnis, insbesondere ein Vlies bevorzugt aus Fasern auf der Basis von Zellulose und / oder Polyesterfasern. Zusätzlich können die Fasern von einem organischen Bindemittel, das bevorzugt Hydroxyl und/oder Estergruppen enthält, zusammengehalten werden. Solche Vliese sind in der deutschen Patentanmeldung DE-OS-3802366 beschrieben.

Bevorzugt werden als Zellulosefasern Zellwolle, Zellstoff oder Linters eingesetzt. Als Zellwolle wird ein Material bezeichnet, das durch Alkalisieren von Zellulose zu Alkali-Zellulose, anschließender Behandlung mit Schwefelkohlenstoff und der Bildung von Zellulose-Xanthogenaten, Auflösung der Zellulose-Xanthogenate in Lauge und Verspinnen von Viskosefilamentgarn erhalten wurde. Zellstoff kann durch einen vollständigen chemischen Aufschluß zellulosehaltiger Materialien und anschließende Bleiche gewonnen werden. Als Linters werden kurze, nicht spinnbare Baumwollfasern bezeichnet, die aus Baumwollsamen erhalten werden. Die Zellulosefasern weisen bevorzugt eine Faserfeinheit von 1,7 bis 4,5 dtex auf und besitzen Längen von 1 bis 20 mm, bevorzugt 3 bis 12 mm. Besonders bevorzugte Polyesterfasern sind Fasern mit einem spezifischen Gewicht von ca. 1,17 g/cm³, einer Länge von 3 bis 6 mm und einer Faserfeinheit von 1,7 bis 3,3 dtex.

Als weiteren Bestandteil können die Vliese ein organisches Bindemittel, das OH- und/oder Estergruppen aufweist, enthalten. Bevorzugt werden hierzu Polyvinylalkohol und Epichlorhydrinharze eingesetzt. Der Polyvinylalkohol wird vorzugsweise als Fasermaterial mit einer Länge von 3-5 mm und einem spezifischen Gewicht von 1,26 bis 1,30 g/cm³ eingesetzt. Aus diesen Komponenten und voll entsalztem Wasser wird ein Vlies auf einer Schrägsiebmaschine nach dem üblichen Verfahren der Papierherstellung erzeugt. Besonders bevorzugte Vliesmaterialien sind außerdem in DE-OS-3802366 beschrieben.

Die Dicke des Materials des Probennahmeelementes ist nicht entscheidend. Vorteilhafterweise sollte es eine möglichst ebene Oberfläche haben, wobei die Dicke üblicherweise zwischen 0,1 und 3 mm liegt. Die Probennahmeoberfläche sollte an die Dimensionen des Chromatographie-Teststreifens angepaßt sein, d. h. die Breite der Probennahmefläche sollte die Breite des Teststreifens nicht wesentlich übersteigen. Bevorzugte Dimensionen der Probennahmefläche liegen zwischen 0,3 und 2 cm, besonders bevorzugt von 0,5 bis 0,8 cm in der Länge und in der Breite von 0,3 bis 1 cm, besonders bevorzugt von 0,4 bis 0,8 cm.

Nach dem Abwischen einer kontaminierten Oberfläche mit einem Probennahmeelement wird die Probennahmeoberfläche dieses Elementes mit einem Bereich der Teststreifenoberfläche zwischen der Elutionsmittelaufgabezone und der Zielzone kontaktiert, bevorzugt leicht aufgedruckt. Dieser Bereich liegt bevorzugt zwischen der Elutionsmittelaufgabezone und der Konjugatzone oder auf der Konjugatzone selbst. Besonders bevorzugt ist außerdem, wenn die Zone, auf der das Probennahmeelement aufgedrückt wird, aus einem der für das Probennahmeelement geeigneten Materialien besteht, insbesondere den in der DE-OS-3802366 beschriebenen Vliesmaterialien. Ganz besonders bevorzugt ist es, wenn das Probennahmeelement auf die Konjugatzone aufgedrückt wird. Bevorzugt ist deshalb auch, daß das Probennahmeelement zwischen 25% und 150% der Fläche, besonders bevorzugt annähernd die gleiche Fläche wie die Konjugatzone hat.

Der Druck, mit dem das Probennahmeelement aufgedrückt wird, sollte mindestens so groß sein, daß ein flächiger Fluidkontakt zwischen beiden Oberflächen möglich ist.

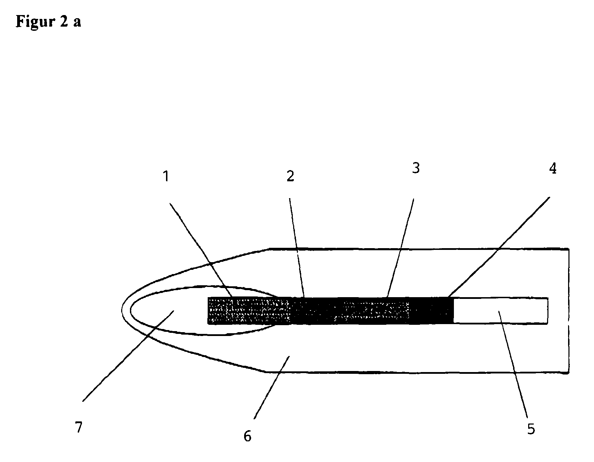

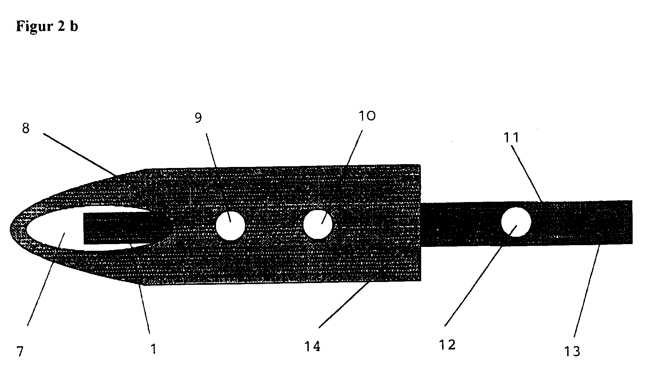

Um dem Benutzer das Aufdrücken zu erleichtern, kann in einer Ausführungsform der Testträger (5) in einem Gehäuse (6,8) untergebracht sein (Figur 2a: Gehäuse ohne Deckel, Figur 2b: Gehäuse geschlossen). Das Gehäuse weist eine Öffnung (9) zum Aufdrücken des Probennahmeelementes (13) auf Das Probennahmeelement (13) ist auf einem Träger (11) so an ein Scharnier (14) des Gehäuses befestigt, daß dieser Träger zum Wischen ausgeklappt ist und zum Aufdrucken des Probennahmeelementes auf die Öffnung (9) des Teststreifengehäuses umgeklappt und gegebenenfalls arretiert werden kann. Das Probennahmeelement kann auch mit der Hand oder einer Klammer mit dem Teststreifen kontaktiert werden.

Im nächsten Schritt wird Elutionsflüssigkeit auf die Elutionsmittelaufgabezone (1) aufgebracht. Die Flüssigkeit kann auf die Elutionsmittelaufgabezone aufgegeben werden oder der Test-streifen in eine Elutionsflüssigkeit getaucht werden. Wird die Flüssigkeit aufgebracht, besteht die Elutionsmittelaufnahmezone bevorzugt aus einem besonders saugfähigen Material, das mindestens so viel Flüssigkeit aufnimmt, daß die Flüssigkeit bis zum Ende des Chromatographie-Streifens wandert. Ist der Testträger in einem Gehäuse, hat das Gehäuse bevorzugt eine Öffnung (7) zur Aufgabe der Flüssigkeit.

Als Elutionsflüssigkeit kommt Wasser und die bei Immunoassays üblichen Pufferlösungen in Frage. Die Flüssigkeit wandert entlang des Streifens in Richtung Zielzone (4) und passiert dabei die Zone mit dem aufgedrückten Probennahmeelement. Überraschenderweise werden dabei auf dem Probennahmeelement haftende Analytmoleküle von dem Flüssigkeitsstrom aufgenommen und in die weiteren Zonen weitertransportiert. Bevorzugt ist, wenn die Zone des Teststreifens, auf die das Probennahmeelements aufgedruckt wird ("Aufnahmezone") aus einem der für das Probennahmeelement bevorzugten Vlies-Materialien besteht. Besonders bevorzugt ist, wenn die Aufnahmezone ("Aufnahmevlies") durch die Konjugatzone (2) selbst gebildet wird.

In einer bevorzugten Testvariante werden Analytmoleküle beim Passieren der Konjugatzone durch markierte Analyt-Bindungspartner komplexiert, nicht-komplexierte markierte Bindungspartner werden in der Abfangzone durch immobilisierte Analyt-Analoga, z. B. Polyhaptene, festgehalten, während markierte Bindungspartner, die einen Analyten gebunden haben, die Abfangzone passieren und die Zielzone erreichen. In der Zielzone können dann die markierten Komplexe detektiert werden. Um das Signal in der Zielzone optisch besser von der Markierung in der Abfangzone unterscheiden zu können, kann die Abfangzone vorteilhafterweise abgedeckt sein. Wenn der Testträger in einem Gehäuse untergebracht ist, hat dieses über der Zielzone bevorzugt eine Öffnung (10) zum Beobachten des Signals.

Die Analyse auf eine Analytkontamination der untersuchten Oberfläche ist dann positiv, wenn mindestens ein Teilbereich der Zielzone eine Färbung aufweist. Die Färbung kann leicht visuell oder photometrisch detektiert werden.

Es ist auch möglich, mit einem Teststreifendevice mehrere Analyten nachzuweisen. Hierzu kann die Konjugatzone und die Abfangzone und gegebenenfalls auch das chromatographische Material von der Konjugatzone bis zur Zielzone in mehrere, in Teststreifenrichtung parallele, vorteilhafterweise voneinander getrennte Teilstreifen unterteilt sein, wobei in einzelnen Teilkonjugatzonen und Teilabfangzonen Bindungspartner für die unterschiedlichen nachzuweisenden Analyten oder unterschiedliche Analytanaloga enthalten sind. Nach Kontaktieren des Probennahmeelementes mit einer gemeinsamen Aufnahmezone vor den Konjugatzonen und Aufgabe der Elutionsflüssigkeit, teilt sich in den verschiedenen Teilkonjugatzonen die Analytflüssigkeit auf verschiedene Teilchromatographiestrecken auf, wobei in jeder Teilzielzone ein anderer Analyt nachgewiesen werden kann.

Die Empfindlichkeit des erfindungsgemäßen Verfahrens ist überraschenderweise wesentlich höher als die der Verfahren aus dem Stand der Technik. Die Wisch- und Übertragungseffizienz des Probennahmeelementes von Analyten auf den Teststreifen ist wider Erwarten so groß, daß mit dem Verfahren Absolutmengen von bis zu 10 ng Analyt, insbesondere Drogen, auf Oberflächen nachgewiesen werden können. Das Verfahren erfordert sehr wenige Handhabungsschritte und das Ergebnis kann sehr schnell und mit einfachen Mitteln ermittelt werden. Besonders überraschend ist es, daß Analyt insbesondere Drogen erfolgreich in Körperflüssigkeiten nachgewiesen werden können, die nur in geringen Mengen auf oder in Körperregionen vorliegen, wie insbesondere Schweiß und Speichel. Durch die Probennahme an Körperregionen, die nicht bei der Handhabung mit dem zu bestimmenden Analyt in Kontakt gekommen sein können, kann nach Vorlage des Untersuchungsergebnisses entschieden werden, ob eine Aufnahme des Stoffes in den Körper vorgelegen hat oder nicht

### Beispiel 1

### a. Herstellung von Benzoylecgoninmaleimidoethylamid

2.4 g Benzoylecgoninhydrochlorid werden in 200 ml trockenem Acetonitril mit 1 g N-Hydroxysuccinimid und 1.8 g Dicyclohexylcarbodiimid versetzt und 3 Std. gerührt. Vom Niederschlag wird abfiltitriert, das Filtrat eingedampft, in Nitromethan aufgenommen und nochmals filtriert. Nach Abdampfen des Lösungsmittels wird mit Ether verrieben. Man erhält 1.13 g Benzoylecgoninsuccinimidylester. Dieses Produkt wird zusammen mit 0.47 g Maleimidoethylaminhydrochlorid (s. WO 90/15798) in 100 ml trockenem Acetonitril aufgenommen. Man gibt 1.1 g Triethylamin zu und rührt 12 Stunden bei Raumtemperatur. Das Reaktionsgemisch wird eingedampft, in 50 ml Essigester aufgenommen und drei Mal mit Natriumhydrogencarbonatlösung ausgeschüttelt. Die Essigesterphase wird eingedampft und das Produkt durch Aufnahme in 10 ml mit HCl gesättigtem Dioxan in das Hydrochlorid überführt. Man filtriert, wäscht mit Ether und erhält so 1 g Benzoylecgoninmaleimidoethylamidhydrochlorid.

### b. Herstellung eines biotinylierten Cocain-Polyhaptens für die Abfangzone

Kaninchen-IgG wird bei einer Konzentration von 25 mg/ml in Phospatpuffer pH 8 mit der 6fach molaren Menge S-Acetylthiopropionsäuresuccinimidylester gelöst in Dimethylsulfoxid umgesetzt. Nach 1 Stunde bei 25 °C wird die Reaktion durch Zugabe einer Lösung von 1 mol/l Lysin gestoppt. Es folgt Dialyse gegen 0.1 mol/l Kaliumphosphatpuffer, pH 6 mit 1 mmol/l EDTA. Anschließend wird der pH auf 7.8 eingestellt und mit 1 mol/l Hydroxylamin-Lösung, pH 7,5 ad 2o mmol/l 1 Stunde bei 25 °C inkubiert. Zur Kopplung wird ein 5fach molarer Überschuß Benzoylecgoninmaleimidoethylamidhydrochlorid in Dimethylsulfoxid gelöst und unter Rühren zu der Lösung des mit Sulfhydrylgruppen modifizierten Kaninchen-IgG gegeben. Nach Inkubation bei 25 °C für 2 Stunden wird die Reaktion gestoppt durch die successive Zugabe von 0.1 mol/l Cystein-Lösung ad 1 mmol/l und 0.5 mol/l Jodacetamidlösung ad 5 mmol/l. Der Ansatz wird über Nacht gegen 0.1 mol/l Kaliumphosphatpuffer, pH 8.5 dialysiert und über Membranfiltration auf eine Proteinkonzentration von 10 mg/ml konzentriert. Danach wird das erhaltene Cocain-Polyhapten mit einem 8fach molarem Überschuß Biotinylcapronsäuresuccinimidylester, gelöst in Dimethylsulfoxid, biotinyliert. Der Ansatz wird gegen 20 mmol/l Natriumacetat, pH 4.3, dialysiert und über FPLC aufgereinigt.

### c. Herstellung von Morphin-3-O-essigsäuremaleimidoethylamidhydrochlorid

Analog Beispiel 1 a. wird Morphin-3-O-essigsäure mit Maleimidoethylaminhydrochlorid zu Morphin-3-O-essigsäuremaleimidoethylamidhydrochlorid umgesetzt.

### d. Herstellung eines biotinylierten Morphin-Polyhaptenes

Analog Beispiel 1 b. wird mit Sulfhydrylgruppen modifiziertes Kaninchen-IgG mit Morphin-3-O-essigsäuremaleimidoethylamidhydrochlorid und Biotinylcapronsäuresuccinimidylester zu einem biotinylierten Morphin-Polyhapten umgesetzt.

### e. Herstellung eines Goldkonjugates aus einem Anti-Cocain-Antikörper

Goldsol mit einem durch Photonen-Korrelations-Spektroskopie bestimmten Partikeldurchmesser von 20 nm wurde nach Standardverfahren (Frens, Nature Physical Science, Vol. 241, S. 20-22, 1973) hergestellt. Die Konjugation mit dem Antikörper, der Cocain und Benzoylecgonin erkennt, wird nach dem Stand der Technik durchgeführt (Geoghegan et al., J. Immunol. Meth. Vol.. 34, S. 11-31, 1980)

### f. Herstellung eines Goldkonjugates aus einem Anti-Morphin-Antikörper

Analog Beispiel 1 e wurde ein Antikörper, der Morphin und Heroin erkennt, an Goldpartikel gebunden.

### Beispiel 2

### a. Testträger zur Bestimmung von Cocain

Aufbau des Testträgers siehe Figur 1.

### Elutionsmittelaufgabezone (Saugvlies) (1)

Polyestervlies der Firma Binzer, Hatzfeld, Bundesrepublik Deutschland. Es handelt sich um ein reines Polyestervlies, das mit 10% Kuralon verfestigt ist. Die Dicke beträgt 1,0 - 1,2 mm, die Saugkapazität 1800 ml/m².

### Konjugatzone (Konjugatvlies) (2)

Ein Mischvlies aus 80 Teilen Polyester und 20 Teilen Zellwolle, verfestigt mit 20 Teilen Kuralon, in einer Dicke von 0,32 mm und mit einer Saugkapazität von 500 ml/m² wird mit folgender Lösung getränkt und anschließend getrocknet: 100 mmol/l HEPES-Puffer pH 7.5, 100 mol/l NaCl, Konjugat aus Goldpartikeln und einem Anti-Cocain-Antikörper, der auch an Benzylecgonin bindet in einer Konzentration, die eine optische Dichte von 10 bei 520 nm aufweist.

### Abfangzone (3)

Ein Vlies aus 100% Linters, verfestigt mit 2% Etadurin mit einer Dicke von 0.35 mm und einer Saugkapazität von 372 ml/m² wird mit folgender Lösung getränkt und anschließend getrocknet: 10 mmol/l Natriumphosphat pH 7.5, polymerisiertes Streptavidin 200 mg/l (Herstellung nach Beispiel 1c, EP A 0 331 127).
Das vorgetränkte Vlies wird anschließend nochmals getränkt und anschließend getrocknet: 10 mmol/l Natriumphosphat pH 7.5, 200 mg/l biotinyliertes Cocain-Polyhapten aus Beispiel 1 b.

### Nachweisfeld (Zielzone) (4)

Es wird ein Vlies aus 100% Linters, verfestigt mit 2% Etadurin mit einer Dicke von 0.35 mm und einer Saugkapazität von 372 ml/m² eingesetzt.

Alle Vliese haben eine Breite von 5 mm. Das Konjuagtvlies hat eine Größe von 5 x 5 mm. Die Vliese werden gemäß Fig. 1 auf eine Trägerfolie (5) von 5 mm Breite geklebt.

### b. Testträger zum Nachweis von Heroin

Analog wird aus dem Goldkonjugat des Anti-Heroin-Antikörpers und dem biotinylierten Morphin-Polyhapten ein Testträger zum Nachweis von Heroin hergestellt.

### Beispiel 3

Auf eine Polyethylenoberfläche werden jeweils 10 µl einer verdünnten Heroinhydrochloridlösung in Methanol aufgetragen und eintrocknen gelassen. Man erhält je mit 10, 20, 40, 60 und 80 ng Heroinhydrochlorid kontaminierte Flächen von ca. 1 cm² Fläche. Von jeder Menge werden drei Testfelder präpariert.

Es wurde ein Vlies hergestellt, das aus 80 Teilen Polyesterfasern einer Faserfeinheit von 3,3 dtex und einer Faserlänge von 4 mm besteht, 20 Teilen Zellwolle mit einer Faserfeinheit von 1,7 dtex und einer Schnittlänge von 3 mm sowie 20 Teilen Polyvinylalkoholfasern einer Schnittlänge von 4 mm. Die Faserstoffe Polyester, Zellwolle und Polyvinylalkohol wurden mit VE-Wasser bei einer Stoffdichte von 0,3% in Mischbütten aufgeschlagen bzw. vereinzelt. Der Faserstoff wurde anschließend auf ein umlaufendes Sieb gepumpt. Während das Fasergemisch entwässert bzw. das Wasser durch Vakuum abgesaugt wird, orientieren sich die Fasern auf der Siebseite und werden als Vlies mit einem Trockengehalt von ca. 20% über Trockenzylinder kontaktgetrocknet. Man erhält ein Vlies mit einem Flächengewicht von 80 g / m² und einer Dicke von 0.32 mm.

Aufeinen Träger (1) werden 5 x 5 mm große Stücke dieses Vlieses (2) aufgeklebt (Figur 3). Mit dem auf den Träger montierten Probennahmevlies werden die mit Heroin kontaminierten Testfelder mit leichtem Druck abgewischt.

Das auf den Träger montierte Probennahmevlies wird über dem Konjugatvlies des nach Beispiel 2 b. hergestellten Testträgers positioniert und mit leichtem Druck aufgedrückt und mit einer Klammer fixiert.

Die Elutionsmittelsaufgabezone wird 5 sec. in einen Chromatographiepuffer (150 mmol/l NaCl, 50 mmol/l Kaliumphosphatpuffer pH 7,2) getaucht. Man legt auf eine nicht saugende Unterlage ab, und bestimmt nach 2 min mit einem Chromameter der Fa. Minolta die Buntheit (C-Wert). Danach wird visuell das Nachweisfeld auf die Anwesenheit von rosa Farbe geprüft.

| Menge Heroin Testfeld [ng] | C-Wert | visuelle Ablesung¹ |
|---|---|---|
| 0 | 3.36 | 0 |
| 10 | 2.99 | 0 |
| 10 | 3.05 | 0 |
| 10 | 3.30 | 0 |
| 20 | 7.88 | + |
| 20 | 9.46 | + |
| 20 | 6.89 | +/- |
| 40 | 5.68 | +/- |
| 40 | 8.74 | + |
| 40 | 11.35 | + |
| 60 | 12.00 | ++ |
| 60 | 10.31 | + |
| 60 | 15.05 | ++ |
| 100 | 14.17 | ++ |
| 100 | 10.26 | + |
| 100 | 16.85 | ++ |

| | | |
|---|---|---|
| ¹0: keine Rosafärbung im Nachweisfeid sichtbar +/- schwache Farbe nachweisbar + Farbe nachweisbar ++ starke Farbe nachweisbar | | |

### Beispiel 4

Mit einer verdünnten Cocainlösung in Wasser wurden analog Beispiel 3 auf eine Polyethylenfolie Testbereiche mit 5, 10, 25, 50, 75 und 100 ng Cocain aufgetragen. Analog Beispiel 3 werden mit einer Vorrichtung nach Figur 2, die jeweils Testträger nach Beispiel 2 a.zur Bestimmung von Cocain enthält die Testbereiche abgewischt. Die Probennahmevliese sind rund bei einem Durchmesser von 4 mm. Das Nachweisfeld wird jeweils visuell auf Rosafärbung geprüft.

| Menge Cocain / Testfeld [ng] | visuelle Ablesung² |
|---|---|
| 0 | 0 |
| 5 | +/- |
| 10 | +/- |
| 25 | + |
| 50 | ++ |
| 75 | ++ |
| 100 | ++ |

| | |
|---|---|
| ²0: keine Rosafärbung im Nachweisfeld sichtbar +/- schwache Farbe nachweisbar + Farbe nachweisbar ++ starke Farbe nachweisbar | |

### Beispiel 5

Ein Teil Cocain wird mit 1000 Teilen Lactose vermischt. 5 mg dieses Gemisches werden auf einer Fläche von 220 cm² eines Baumwolltuches verteilt. Ca. 10 cm² diese Bereiches werden anaolg Beispiel 4 abgewischt und analysiert. In allen Versuchen wird eine Rosafärbung in dem Nachweisfeld visuell detektiert. Analog wird eine Polyethylenfolie von 2 cm² mit cocainhaltigen Partikeln kontaminiert und durch Wischen analysiert. Es wird ebenfalls in allen Versuchen wird eine Rosafärbung in dem Nachweisfeld visuell detektiert.

### Beispiel 6

Auf eine rauhe anodisierte Aluminiumplatte werden je 10 µl entsprechend verdünnter Lösungen von Cocainhydrochlorid in Wasser aufgegeben und auf eine Fläche von 1 cm² verteilt. Nach Antrocknen bei 37 °C für 15 min. werden die kontaminierten Flächen mit einem runden Vlies nach Beispiel 3 mit einem Durchmesser von 5 mm mit leichtem Druck abgewischt. Dabei wird sowohl ohne Befeuchtung (trocken) als auch nach vorheriger Befeuchtung des Probennahmevlieses mit 2 µl Wasser gewischt. Die Probennahmevliese werden dann jeweils auf die Konjugatzone eines Testträgers zum Nachweis auf Cocain nach Beispiel 3 aufgelegt und mit einer flachen Pinzette festgehalten. Das Saugvlies des Testträgers wird zur Hälfte für 10 sec. in Wasser getaucht. Anschließend legt man den Testträger auf einer flachen nicht saugenden Unterlage ab und bestimmte nach 2 min in dem Nachweisfeld die Farbe.

| Absolutmenge Cocainhydrochlorid im Testfeld [ng] | visuelle Ablesung nach Wischen mit trockenem Wischvlies³ | visuelle Ablesung nach Wischen mit feuchtem Wischvlies |
|---|---|---|
| 1000 | ++ | ++ |
| 600 | ++ | ++ |
| 300 | + | ++ |
| 200 | + (schwach) | ++ |
| 100 | 0 | ++ |
| 60 | 0 | ++ |
| 30 | 0 | ++ |
| 25 | 0 | ++ |
| 16 | 0 | ++ |
| 8 | 0 | + |
| 4 | 0 | 0 |

| | | |
|---|---|---|
| ³0: keine Rosafärbung im Nachweisfeld sichtbar +: Rosafärbung im Nachweisfeld sichtbar ++: starke Rosafärbung im Nachweisfeld sichtbar | | |

### Beispiel 7

Bei der Untersuchung der Körperoberfläche von Personen zeigt sich folgendes Bild: Bei der Untersuchung der Körperoberfläche unter der Achsel wird mit dem Testträger bei Personen, die Opiate bzw. Kokain konsumiert hatten (nachgewiesen durch einen positiven Befund im Urin) ein positives Ergebnis erhalten. Der Ort auf der Körperoberfläche schließt aus, daß es sich bei den nachgewiesenen Analyten um Drogenmoleküle handelt, die durch externe Kontamination auf die Körperoberfläche gelangt sind. Die nachgewiesenen Drogenmoleküle bzw. deren Metaboliten sind mit Hilfe von Schweiß abgelagert worden, das heißt, sie müssen konsumiert worden sein.

| | Kokain | | Opiate | |
|---|---|---|---|---|
| Proband Nr. | Ergebnis mit Verfahren | Urinbefund | Ergebnis mit Verfahren | Urinbefund |
| 1 | - | - | + | + |
| 2 | + | + | + | + |
| 3 | - | - | + | + |
| 4 | - | - | - | - |
| 5 | - | - | - | - |

## Patentansprüche

1. Verfahren zum Nachweis eines Analyten in Körperflüssigkeit mit einem Testkit, das umfaßt,
a) einen Teststreifen aus einem oder mehreren kapillaraktiven chromatographiefähigen flächenförmigen Materialien in Fluidkontakt zueinander mit
- einer Elutionsmittelaufgabezone an einem Ende und einer Zielzone am anderen Ende,
- einer Abfangzone zwischen Elutionsmittelaufgabezone und Zielzone, in der ein Abfangreagenz immobilisiert ist, das fähig ist, entweder den Analyten, einen spezifischen Analytbindungspartner oder einen markierten Bindungspartner spezifisch zu binden und
- einer Konjugatzone, zwischen Elutionsmittelaufgabezone und Abfangzone, die einen wanderungsfähigen markierten Bindungspartner enthält, der fähig ist, entweder den Analyten oder den spezifischen Analytbindungspartner oder das Abfangreagenz spezifisch zu binden, wobei die Bindung des markierten Bindungspartners zu einem detektierbaren Signal in der Abfangzone oder in der Zielzone führt, das die Anwesenheit des Analyten anzeigt,
b) ein Probennahmeelement separat zur Teststreifenoberfläche
c) eine Vorrichtung, die bewirkt, daß das Probennahmeelement mit der Teststreifenoberfläche zwischen Elutionsmittelauftragszone und Konjugatzone oder in der Konjugatzone selbst kontaktiert werden kann,
dadurch gekennzeichnet, daß mit dem Probennahmeelement Körperflüssigkeit von einer Körperregion aufgenommen und untersucht wird.

2. Verfahren gemäß Anspruch 1, dadurch gekennzeichnet, daß
das Probennahmeelement mit der Oberfläche des Teststreifens kontaktiert wird, Elutionsflüssigkeit auf die Elutionsmittelaufgabezone aufgegeben wird, die in Richtung zur Zielzone an der Kontaktstelle mit dem Probennahmeelement vorbeiwandert, wobei der Analyt von der Elutionsflüssigkeit aufgenommen wird und
die Anwesenheit des Analyten in der Zielzone aufgrund einer immunologischen Bindungsreaktion gemessen wird.

3. Verfahren gemäß Anspruch 1, dadurch gekennzeichnet, daß der nachzuweisende Analyt eine illegale Droge oder deren Metabolit ist.

4. Verfahren gemäß einem der Ansprüche 1-3, dadurch gekennzeichnet, daß es sich bei der Körperregion um Haut oder Schleimhaut handelt.

5. Verfahren gemäß einem der Ansprüche 1-4, dadurch gekennzeichnet, daß es sich bei der Körperflüssigkeit um eine Körperausscheidung handelt.

6. Verfahren gemäß Anspruch 5, dadurch gekennzeichnet, daß die Körperausscheidung Schweiß oder Speichel ist.

7. Verfahren gemäß Anspruch 1, dadurch gekennzeichnet, daß das Material des Probennahmeelementes mit der Andruckvorrichtung fest verbunden ist.

8. Verwendung eines Teskits umfaßend
a) einen Teststreifen aus einem oder mehreren kapillaraktiven chromatographiefähigen flächenförmigen Materialien in Fluidkontakt zueinander mit
- einer Elutionsmittelaufgabezone an einem Ende und einer Zielzone am anderen Ende,
- einer Abfangzone zwischen Elutionsmittelaufgabezone und Zielzone, in der ein Abfangreagenz immobilisiert ist, das fähig ist, entweder den Analyten, einen spezifischen Analytbindungspartner oder einen markierten Bindungspartner spezifisch zu binden und
- einer Konjugatzone, zwischen Elutionsmittelaufgabezone und Abfangzone, die einen wanderungsfähigen markierten Bindungspartner enthält, der fähig ist, entweder den Analyten oder den spezifischen Analytbindungspartner oder das Abfangreagenz spezifisch zu binden, wobei die Bindung des markierten Bindungspartners zu einem detebierbaren Signal in der Abfangzone oder in der Zielzone führt, das die Anwesenheit des Analyten anzeigt,
b) ein Probennahmeelement separat zur Teststreifenoberfläche
c) eine Vorrichtung, die bewirkt, daß das Probennahmeelement mit der Teststreifenoberfläche zwischen Elutionsmittelauftragszone und Konjugatzone oder in der Konjugatzone selbst kontaktiert werden kann,
zur Bestimmung eines Analyts in einer Körperflüssigkeit, wobei diese Körperflüssigkeit von einer Körperregion aufgenommen wird.
